# EUROPÄISCHE PATENTSCHRIFT

(11) **EP 2 797 570 B1**
(45) Veröffentlichungstag und Bekanntmachung des Hinweises auf die Patenterteilung: **05.12.2018**
(21) Anmeldenummer: 12809791.2
(22) Anmeldetag: 19.12.2012
(51) Int. Cl.: A61Q 15/00, A61K 8/891, A61K 8/34, A61K 8/36, A61K 8/892, A61K 8/92, A61K 8/26, A61K 8/37, A61K 8/41, A61K 8/04

(54) **Verwendung zur verringerten Fleckenbildung und verbesserten Auswaschbarkeit**
Use for reduced staining and improved washability
Utiliser pour réduire les taches et améliorer la lavabilité

(30) Priorität: 29.12.2011 DE 102011090113
(43) Veröffentlichungstag der Anmeldung: 05.11.2014
(73) Patentinhaber: Beiersdorf AG, 20253 Hamburg (DE)
(72) Erfinder: WINDISCH, Björna, 22607 Hamburg (DE); BÜRGER, Anette, 22529 Hamburg (DE); BLUSCHKE, Torsten, 21717 Fredenbeck-Schwinge (DE); Scholz, Annika, 22844 Norderstedt (DE); Urban, Michael, 22534 Hamburg (DE)
(74) Vertreter: Hartmann, Jost
(86) Internationale Anmeldenummer: PCT/EP2012/076058
(87) Internationale Veröffentlichungsnummer: WO 2013/098141

(56) Entgegenhaltungen:
- WO-A2-2010/097205
- WO-A2-2012/085055
- JP-A- 03 095 111
- DATABASE GNPD [Online] MINTEL; November 2011 (2011-11), "UNISEX DEODORANT SPRAY", XP002714550, Database accession no. 1659454
- DATABASE GNPD [Online] MINTEL; Mai 2009 (2009-05), "SPRAY DEODORANT", XP002714551, Database accession no. 1090048

## Beschreibung

Die Erfindung umfasst die Verwendung von Silikon-Gums in antitranspirantwirksamen Zubereitungen zur Verminderung der Fleckenbildung in oder auf der Kleidung und der Verbesserung der Auswaschbarkeit von Flecken aus der Kleidung.

Antitranspirantien oder Desodorantien dienen dazu, Körpergeruch zu beseitigen oder verhindern, der entsteht, wenn der an sich geruchlose frische Schweiß durch Mikroorganismen zersetzt wird. Den üblichen kosmetischen Desodorantien liegen unterschiedliche Wirkprinzipien zugrunde. Im allgemeinen Sprachgebrauch erfolgt nicht immer eine klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruches (Geruchsverbesserungsmittel). Gängige Wirkmechanismen hierzu sind antibakterielle Effekte, wie sie auch das nicht-kolloidale Silber zeigen, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Umsetzung zu wohlriechenden Stoffen umgesetzt werden.

So durch Anwendung eines Mittels kein Einfluss auf die Schweißsekretion ausgeübt wird, also keine antitranspirante Wirkung realisiert wird, liegt erfindungsgemäß kein Antitranspirant vor. Erfindungsgemäß sind demnach als Antitranspirantwirkstoffe solche Stoffe umfasst, die einen Einfluss auf die Schweißsekretion haben.

Die gewünschte Minimierung der Schweißsekretion kann durch verschiedene Mechanismen realisiert werden. Hierzu zählt traditionell der Einsatz von Adstringenzien, welche Eiweißfällungen und Gerinnungen hervorrufen und so eine Verengung oder Verschluss der Schweißdrüse bewirken. Neuartige AT-Wirker basieren bspw. auf dem Prinzip der Anticholinergika, welche die Nervenreize, die zur Sekretionssteigerung der Schweißdrüsen führen, unterbrechen. Ein weiteres Prinzip neuartiger AT-Wirker beruht auf der Beeinflussung von Membrantransportvorgängen in der Zelle. So hemmen spezifische Aquaporin-Inhibitoren die Proteine, die Kanäle in der Zellmembran bilden, um den Durchtritt von Wasser und weiteren Molekülen zu erleichtern. Auch lonenkanal-Hemmer bewirken eine Beeinflussung von Membrantransport- bzw. Osmosevorgängen. Ein weiterer neuartiger AT-Wirkmechanismus lässt sich durch die Verwendung kurzkettiger, vicinaler Diole realisieren, welcher möglicherweise auf deren osmotischen Aktivität zurückzuführen ist.

Üblicherweise werden Antitranspirantien (AT) und Desodorantien (Deo) in mannigfaltigen Produktformen angeboten, wobei in Europa Roller, Pumpzerstäuber und Aerosole dominieren, in den USA, Mittel- und Südamerika eher die Stifte. Es sind sowohl wasserfreie (Suspensionen) als auch wasserhaltige Produkte (hydro-alkoholische Formulierungen, Emulsionen) bekannt.

Bevorzugte Applikationsform für die AT-Formulierungen sind wasserfreie Aerosole, die in Aluminium- oder Weißblechdosen auf den Markt kommen. Diese Applikationsform stellt weltweit die häufigste Form im Bereich Deo/AT dar.

Wasserfrei bedeutet, dass der Anteil an Wasser in den Aerosolzubereitungen, abgesehen von ggf. mit eingeschleppten Verunreinigungen oder Kristallwasser, nahezu 0 Gew.% beträgt.

Ein Aerosol ist ein disperses System, bei dem ein Feststoff und/oder eine Flüssigkeit in einem Gas äußerst fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Gemisch aus Treibmittel und Wirkstofflösung durch eine feine Düse, das Treibmittel verdampft und hinterlässt das fein verteilte Sprühgut als Aerosol. Im anwendungstechnischen Sprachgebrauch wird der Begriff Aerosol vom Fachmann häufig auch im Sinne von Aerosolsprays verwendet, d.h. hier wird unter "Aerosol" nicht der reine Sprühnebel, sondern eine Druckgasverpackung nebst Abgabe- bzw. Dosiervorrichtung und Füllgut verstanden. Das Füllgut umfasst Wirkstofflösung und Treibmittel. Als Wirkstofflösung wird die Summe aller Zubereitungsbestandteile ohne das Treibmittel bezeichnet. Der Begriff Wirkstofflösung wird auch dann verwendet, wenn die Zubereitung z.B. in Form einer Suspension oder Emulsion vorliegt. Als Suspension ist eine Dispersion zu verstehen, bei der die kontinuierliche Phase im flüssigen und die dispergierte Phase im festen Zustand vorliegen.

Unter Aerosolzubereitung bzw. Aerosolprodukt versteht man jeden Behälter aus Metall, Glas oder Kunststoff, einschließlich des darin enthaltenen verdichteten, verflüssigten oder unter Druck gelösten Gases mit oder ohne Flüssigkeit, Paste oder Pulver, der mit einer Entnahmevorrichtung versehen ist, die es ermöglicht, seinen Inhalt in Form von in Gas suspendierten festen oder flüssigen Partikeln als Schaum, Paste, Pulver oder in flüssigem Zustand austreten zu lassen.

Bei Anwendung von Antitranspirantien oder Deodorantien reklamieren viele Verbraucher unerwünschte Flecken im Achselbereich in der Kleidung. Es handelt sich dabei häufig um gelbliche Flecken, die auch zu Verkrustungen neigen können. Diese Ablagerungen und Flecken entstehen vor allem durch ein komplexes Zusammenspiel zwischen Produkt, Hautfett, Schweiß und Waschmittel und lassen sich durch herkömmliche Reinigungsverfahren nur schwer entfernen.

Diese Flecken sind nicht die als "Weißeln" bezeichneten Rückstände auf der Haut oder Kleidung zu verstehen.

Die Flecken können je nach Person unterschiedlich ausgeprägt sein. Eine Ursache sind die in den meisten Deo-AT-Produkten eingesetzten Aluminiumsalze, die als Antitranspirantwirkstoffe fungieren. Diese hartnäckigen Verfärbungen lassen sich beim Waschen, auch bei Vorbehandlungen mit Fleckenmittel, nicht oder nur schwer vollständig entfernen.

Es gibt zahlreiche Literatur und Patente, die sich mit der Fleckenbildung auf der Haut und der Kleidung und deren Vermeidung bei der Anwendung von Antitranspirantien befassen.

Die EP 973492 A1 beschreibt die Verwendung von oberflächenaktiven Substanzen, hauptsächlich nichtionische Emulgatoren, in Antitranspirantien. Die Antitranspirantstiftformulierungen umfassen nichtflüchtige Emollients, ein Träger, z.B. Cyclomethicon, ein Fettalkohol, wie Stearylalkohol, ein Antitransspirantmaterial und ein Tensid. Es wird die Problematik der Bildung von weißen Rückständen auf der Haut und der Kleidung, die sich bei Anwendung von aluminiumhaltigen Antitranspirantien bilden können, beschrieben. Hierin wird das Problem des Weißelns der Formulierung durch einen Refraktionsindexabgleich der Bestandteile angegangen.

In der EP 1178775 A1 wird die Verwendung von wasserlöslichen Tensiden zur Verbesserung der Abwaschbarkeit der Rückstände von der Haut und Kleidung beschrieben. Es werden Kombinationen von adstringierenden Salzen mit wasserlöslichen, nichtionischen Tensiden beschrieben, die einen raschen Antitranspirantwirkungseintritt und eine hohe Effektivität aufweisen sollen.

EP 858317 A1 beschreibt Zubereitungen mit oberflächenaktiven Substanzen mit einem HLB > 10 zur Entfernung der fettigen Rückstände auf der Haut.

EP 696188 A1 beschreibt den Einsatz eines wash-off Agents zur Entfernung der Lipidkomponenten von der Haut, bevorzugt werden dafür Ethoxylate eingesetzt.

Die DE 102008052748 A1, eine frühere Anmeldung der Patentinhaberin, beschreibt, dass in wasserfreien Suspensionen Emulgatoren zur verbesserten Abwaschbarkeit der Formulierung von der Haut eingesetzt werden. Die in der wasserfreien Formel vorteilhaft enthaltenen Strukturgeber können auf der Haut fühlbar wachsige Rückstände hinterlassen. Durch die Anwesenheit von polaren Gruppen an den eingesetzten Emulgatoren wird beim Abwaschen der Formulierung die Affinität zum Wasser erhöht und die Rückstände verschwinden. Dafür eignen sich bevorzugt nichtionische Emulgatoren.

Die WO 2011050044 A1 beschreibt Zubereitungen zur Reduzierung oder Eliminierung gelber Flecken mit einem AT-Mittel und mindestens einem Antioxidans.

Die WO 2010097205 A2 beschreibt den Einsatz von geladenen Tensiden zur Verringerung der Textilverfleckung.

Nachteilig ist der zwingende Zusatz an Tensiden, die galenisch ggf. Probleme mitverursachen können.

Wünschenswert ist es, kosmetische Antitranspirantien zur Verfügung zu stellen, die eine verringerte Fleckenbildung in der Kleidung aufweisen und vor allem die nachträgliche Auswaschbarkeit verbessern helfen ohne den Zusatz an Tensiden.

Die kosmetischen Zubereitungen sollen darüber hinaus keinerlei Instabilitäten aufweisen und sollten einfach zu formulieren sein.

Wesentlicher Aspekt bei der Formulierung kosmetischer Antitranspirantien ist deren Hautverträglichkeit, so dass neben der Aufgabe der Fleckvermeidung oder verbesserten Auswaschbarkeit auch die Hautverträglichkeit zu berücksichtigen ist.

Im Bereich der antitranspirantwirksamen Aerosolsprays ist der Gebrauch verschiedener Silikonöle weit verbreitet. Zu den silikonhaltigen Ölen zählen auch die als Silikon-Gums bezeichneten hochmolekularen Polydiorganosiloxane. Silikon-Gums sind beispielsweise in der US 4152416 beschrieben. Hochmolekular bedeutet eine Molmasse von ca. 100.000 bis 2.000.000 g/mol. Bekannte Silikon-Gums sind polymere Polydimethylsiloxane (INCI-Bezeichnung: Dimethicone), z. B. die unter der Bezeichnung DC 1411 Fluid und DC 1413 Fluid von Dow Corning vertriebenen Produkte, und Dimethylsiloxane mit Hydroxy-Endgruppen (INCI-Bezeichnung: Dimethiconol), z. B. die Handelsprodukte DC 1501. Für kosmetische Anwendungen weitere bekannte Silikon-Gums sind beispielsweise SF1236, SF1276, CF1251 von Momentive Performance Materials.

Bekannt sind des Weiteren die Silikon-Gums der Fa. Dow Corning mit den Bezeichnungen Dow Corning® 1501 Fluid (Cyclopentasiloxane, Dimethiconol), Dow Corning® 1503 Fluid (Dimethicone, Dimethiconol), Dow Corning® CB 1556 Fluid (Phenyl Trimethicone, Dimethiconol) und Dow Corning® BY 25-320 (C13-16 Isoparaffin, Dimethicone). Mischungen von Silikonflüssigkeiten und flüchtigen Silikonölen, wie Cyclomethicone, und Silikon-Gums sind ebenfalls bekannt in kosmetischen Zubereitungen. Das im Handelsprodukt DC 1503 zu 88% enthaltene Dimethicone hat ein Molekulargewicht unter 100.000 g/mol und ist somit kein erfindungsgemäßes Silikon-Gum .

WO 9804236 A1 beschreibt Antitranspirantien (AT) mit Silikonlatex und weiteren Verdickern, wie u.a. Silikon-Gum .

EP 452762 A2 beschreibt AT-Aerosole umfassend Silikongummi und Silica, Lösemittel, AT-Salz und ein Treibmittel, wobei eine Kombination von Silica und Silikongummi als Suspendierhilfsmittel eingesetzt wird.

WO 2005074877 A1 beschreibt Aerosol-Antitranspirantien mit gegenüber den AT-Mitteln inerten Silikonen.

WO 2005063188 A1 beschreibt silikonfreie AT-Sprays.

DE 102005047370 A1 offenbart AT-haltige W/O-Emulsionen (Sprays), bei denen das AT-Mittel gelöst in der inneren Phase vorliegt.

DE 69823490 T2 beschreibt wasserfreie AT-Aerosole mit 5-25% Pentan und Silikongummi, welches zur Verstärkung der kühlenden Eigenschaften eingesetzt wird.

US 4152416 A lehrt AT-Aerosole mit geringer Nebel- und Staubbildung mit AT-Salz, Treibgas, Suspendierhilfe und einem synthetischen Polymergummi (500.000 bis 100 Mio. cSt).

EP 343843 B1 beschreibt AT-Aerosole mit einer Sprührate von nicht mehr als 0,5 g/s mit AT-Salz, Treibgas und einer Kombination aus Silikonpolymer und einer flüchtigen niedrigviskosen Flüssigkeit.

EP 1803660 A1 offenbart ein kosmetisches Produkt, das eine Wasser-in-ÖI-Emulsion mit mindestens einem kosmetischen oder dermatologischen Wirkstoff, mindestens ein Treibmittel und eine Aerosol-Abgabevorrichtung umfasst, wobei die mit der Emulsion in Kontakt kommenden Teile des Ventils der Abgabevorrichtung aus nicht-metallischen Materialien bestehen und die Emulsion mit einer Sprührate von 0,05 - 0,5 g/s, bevorzugt 0,1 - 0,35 g/s, besonders bevorzugt 0,15 - 0,2 g/s, ausgestoßen wird.

WO 2010/054921 A1 offenbart eine Aerosolformulierung mit einem Silikongummi.

Es wurde nun überraschend gefunden, dass Silikon-Gums, wie insbesondere Dimethiconol oder Dimethicone, in antitranspirantwirksamen Aerosol-Zubereitungen zu einer signifikanten Fleckreduzierung führen, die ansonsten üblicherweise von dem kosmetischen Aerosolprodukt ohne Silikon-Gums in der Kleidung hervorgerufen worden wären.

Erfindungsgemäß werden daher Silikon-Gums in kosmetischen oder dermatologischen Zubereitungen umfassend ein oder mehrere antitranspirantwirksame Stoffe zur Verminderung oder Vermeidung der Fleckenbildung in oder auf der Kleidung verwendet.

Als Silikon-Gums gelten erfindungsgemäß Polydiorganosiloxane, Dimethicone und Dimethiconol mit einer Molmasse von 100.000 bis 2.000.000 g/mol.

Bevorzugt werden als erfindungsgemäße Silikon-Gums gewählt:
- DC 1501 Fluid (INCI: Cyclopentasiloxane (and) Dimethiconol) 15% Dimethiconol (Aktivgehalt), 85% Cyclopentasiloxane
- DC 1411 Fluid (INCI: Cyclopentasiloxane (and) Dimethicone) 15% Dimethicone (Aktivgehalt), 85% Cyclopentasiloxane.
- DC 1503 Fluid (INCI: Dimethicone (and) Dimethiconol) 12% Dimethiconol (Aktivgehalt), 88% Dimethicone
- DC BY 25-320 (INCI: C13-16 Isoparaffin (and) Dimethicone (and) C10-13 Isoparaffin) 20% Dimethicone Gum (Aktivgehalt), 80% Isoparaffin
- DC 1413 Fluid (INCI: Dimethicone) 14% Aktivgehalt Dimethicone Gum

Erfindungsgemäß geht es um die bekannte gelbliche Verfleckung von Antitranspirantien in oder auf der Kleidung, nachdem das Kleidungsstück gewaschen wurde. Erfindungsgemäß setzt hier auch der Lösungsgedanke der verbesserten Auswaschbarkeit an. Der gelb-Wert des Fleckes wird daher insbesondere über den b*-Wert definiert, der photometrisch mittels der Farbmaßzahlen im CIE- L*a*b-Farbraum ermittelt werden kann.

Daneben kann natürlich auch durch eine einfache Inaugenscheinnahme und Vergleich der erfinderische Verwendungszweck der Verminderung der gelben Flecken festgestellt werden.

Bevorzugt bezieht sich die erfindungsgemäße Verwendung der Fleckenvermeidung bzw. verbesserten Auswaschbarkeit auf Textilien, die Baumwolle enthalten oder aus Baumwolle bestehen.

Erfindungsgemäß bevorzugt, kann die Verwendung von Si-Gums auch mit anderen im Stand der Technik beschriebenen Angängen zur Verringerung der Textilverfleckung kombiniert werden. Dies kann dann zu einer weiteren verbesserten verringerten Fleckenbildung beitragen.

Erfindungsgemäß ist aber allein der Zusatz von Silikon-Gums zur Verringerung der Fleckenbildung und Verbesserung der Auswaschbarkeit der Flecken verantwortlich, wie durchgeführte Untersuchungen belegen.

Unter Verminderung bzw. Vermeidung der Fleckenbildung auf Textilien vor und insbesondere nach der Wäsche wird erfindungsgemäß der verringerte b*-Wert verstanden, der photometrisch mittels der Farbmaßzahlen im CIE- L*a*b-Farbraum ermittelt wird, und der gegenüber den b*-Werten der Textilie, befleckt mit einer Zubereitung mit Antitranspirantmittel aber ohne erfindungsgemäße Si-Gums, gemessen wird.

Nachteilig bei der Verwendung von Antitranspirantien, insbesondere Aluminiumsalzen, ist, wie zuvor ausgeführt, die Bildung von Rückständen in oder auf der Kleidung, die die Kleidung in unschöner Weise verfärben können.

Unter Textilverfleckung werden Verfleckungen, insbesondere im Achselbereich, verstanden. Es handelt sich um Flecken, die nach dem Tragen und/oder Waschen in der Kleidung verbleiben und mit zunehmendem Alter des Kleidungsstückes intensiver werden können. Diese Flecken sind nicht die als "Weißeln" bezeichneten Rückstände auf der Haut oder Kleidung zu verstehen.

Erfindungsgemäß nicht gemeint sind Verfleckungen, die als weiße Flecken im Falle des direkten Kontaktes eines Deos oder Antitranspirants mit dem Stoff entstehen. Dies sind eher die weißlichen Ablagerungen der Rezepturbestandteile, z.B. Aluminiumsalze. Diese Flecken sind leicht vermeidbar, wenn dem Produkt vor Anlegen der Kleidung die Möglichkeit zum Trocknen gegeben wird. Diese weißen Rückstände sind in der Regel mechanisch (Bürsten) oder durch Waschen zu entfernen. Diese Problematik des "Weißelns" ist im Stand der Technik ausführlich beschrieben und es werden darin Lösungsansätze geboten.

Es handelt sich erfindungsgemäß vielmehr um meist gelbliche Verfleckungen, die entstehen, wenn das kosmetische Produkt oder Bestandteile davon beim Schwitzen zusammen mit den Körpersekreten der Achsel auf die Textilie gelangt. Durch das Waschen der Textilie wird ein Teil dieser Ablagerungen ausgewaschen, ein anderer Teil verbleibt als Rückstand auf dem Textil.

Erfindungsgemäß vermag die Zubereitung nicht die Bildung jeglicher Flecken, die sich auf einem Kleidungsstück durch vielfältige Ursachen bilden können, vermindern oder verhindern. Jedoch kann die erfindungsgemäße Zubereitung die Bildung derjenigen Flecken, die durch die Zubereitung selber mit verursacht werden, insbesondere diejenigen durch Antitranspirantstoffe, vermindern oder verhindern sowie deren Auswaschbarkeit verbessern.

Als Maß der Verbesserung bzw. Verminderung wird dabei der Unterschied zur Fleckenbildung bzw. dessen Auswaschung bei der Anwendung der erfindungsgemäßen Zubereitung und der Zubereitung ohne Si-Gum definiert.

Aber auch grundsätzlich beurteilen unabhängige Beobachter (Panel), dass die Flecken der erfindungsgemäßen Zubereitungen auf der Kleidung gar nicht oder zumindest weniger gelb ausfallen. Eine grundsätzliche Fleckenverminderung ist damit gegeben.

Eine Methode zur Überprüfung der durch antitranspirantwirksame Substanzen enthaltende kosmetische oder dermatologische Zubereitungen mitverursachten Flecken auf oder in Kleidung wird in WO 2010097205 A1 beschrieben.

Zur Applikation des Produkt-Sebum-Gemisches kann es vorteilhaft sein, die Probe mittels Siebdruck auf das Prüftextil zu applizieren.

Die Vergleichstests sollten sich dabei auf die den AT-Wirkstoff enthaltene Wirkstofflösung beziehen und nicht auf die versprühte Zubereitung. Bekanntermaßen haben verschiedene Faktoren einen Einfluss auf das Sprühbild der Aerosolzubereitung. Auslassöffnungen, Ventildimensionierung aber auch Bestandteile der Zubereitung können das Sprühbild, den Sprühkegel beeinflussen. Bei einem sehr engen Sprühkegel sind daher folglich mehr "fleckenverursachende" Bestandteile auf dem Testareal der Textilie zu finden als bei Sprühkegeln mit breiterer Streuung, starken Verwirbelungen, Nebelbildung etc.

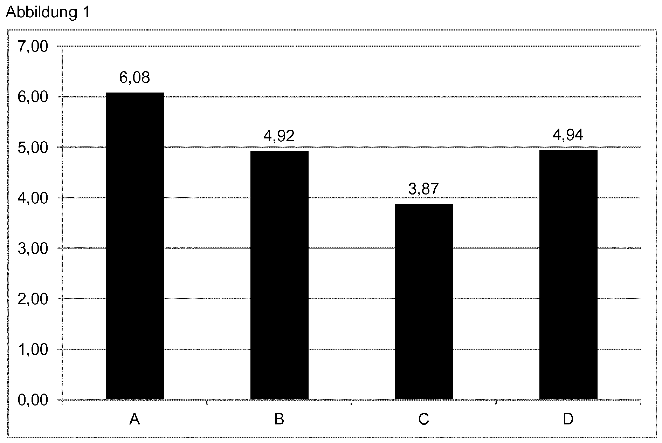

Als Beleg der verbesserten Auswaschbarkeit und verminderten Fleckenbildung der erfindungsgemäßen Zubereitungen wurden in-vitro Untersuchungen durchgeführt, deren Ergebnisse in der Abbildung 1 und Tabelle 1 dargestellt sind.

Es wurden verschiedene Wirkstofflösungen von AT-Spray-Formulierungen hinsichtlich der Bildung von gelben Flecken über drei in vitro Auftragungs-/Waschzyklen untersucht. Es wurde dabei weißes Standard Baumwoll (CO) Köpergewebe verwendet. Die Antitranspirant-Formulierungen wurden im Verhältnis 5:1 mit Sebum nach Bey homogen vermischt und jeweils 5 Testlappen mittels Siebdruckverfahren (TW 24 gem. Institut Hohenstein, 2 Rakelvorgänge) appliziert und direkt im Anschluss im Klimaschrank über Nacht inkubiert (Temperatur: 38 ± 1°C / relative Luftfeuchte: 80 ± 2 %). Nach dem Inkubationsvorgang, der der Simulation der Tragebedingungen dient, erfolge eine Haushaltsmaschinenwäsche (60 °C, Baumwollprogramm, Pulver-Vollwaschmittel, ca. 3 kg saubere Beiladung) und im Anschluss Tumbler-Trocknung. Nach dem 3. Zyklus erfolgte eine farbmetrische Charakterisierung der entstandenen Verfleckung durch Messung der Farbdifferenzen der Fleckstelle zu einer freien Stelle des Testlappens mit dem Farbmessgerät Spectralflash 600 (Datacolor International), Farbmess-Software DatacolorTools, Version 1.1.1, Messgeometrie: d/8°, Glanzkomponente ausgeschlossen, Lichtart: D65 (entsprechend mittlerem Tageslicht), Kalibrierstandard: DCI-Kachel 10848 (Sekundärstandard rückführbar auf NRC (NRC-Report No. PAR-2007-2573 vom 27.11.2007), Messöffnung: 30 mm Durchmesser, Probenlage: 2-lagig, Probenhintergrund: 10 Lagen Unterlagepapier, Prüfklima: 21 °C (± 1°C), 41 % (±4 %) rel. Feuchte.

Zur Auswertung wurde die Veränderung des b-Wertes aus dem CIE-Lab System herangezogen. Die B-Achse charakterisiert im CIE-Lab System den Farbeindruck gelb-blau, wobei positive B-Werte für eine Zunahme des Gelbanteils stehen. Je höher der B-Wert, desto größer ist der Gelbeindruck.

Das CIE-Lab System oder L*a*b*-Farbraum ist ein Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert. Die entsprechende deutsche Norm ist DIN 6174: "Farbmetrische Bestimmung von Farbmaßzahlen und Farbabständen im angenähert gleichförmigen CIELAB-Farbenraum". Die Koordinaten der CIELAB-Ebene werden gebildet aus dem Rot/Grün-Wert a und dem Gelb/Blau-Wert b. Die Lightness-Achse L steht senkrecht auf dieser Ebene.

Nach DIN 6174 sind L, a und b mit * zu schreiben, um sich gegen andere, z.B. das "Hunter-Lab"-System abzugrenzen.

In den Untersuchungen zeigte sich, dass bei beispielsweise 0,5 Gew.% (0,07 % Aktivgehalt) eines Silikon-Gums, bezogen auf die Gesamtmasse der Zubereitung, in der Lage ist, bei einem marktüblichen Antitranspirant Spray die Fleckenbildung (b*, Gelbwert) auf weißer Baumwolle signifikant zu verringern.

**Tabelle 1: getestete Zubereitungen und deren Gelbwert von Flecken; b* - Wert**

| INCI | A | B | C | D |
|---|---|---|---|---|
| | Standard AT-Spray | | | |
| Aluminum Chlorohydrate (Activated) | 5.25 | 5.25 | 2,5 | 5.25 |
| Cyclomethicone | ad 100 | ad 100 | ad 100 | ad 100 |
| Dimethicone (86% M<100,000) + Dimethicone (14%) | --- | 0.5 (0.07 % Aktivgehalt Silikon-Gum) | 0,8 (0,112) | 0.5 (0.07 % Aktivgeha It Silikon-Gum) |
| Octyldodecanol | 0,1 | 0,1 | 0,1 | 0,1 |
| Dimethicone | 0.45 | 0.45 | --- | 0.45 |
| Isopropyl Palmitate | --- | 1,5 | 1,5 | --- |
| C12-15 Alkyl Benzoate | --- | --- | 1,5 | --- |
| Parfum | 1,0 | 1,0 | 1,0 | 1,0 |
| Propylene Carbonate | --- | 0,075 | 0,1 | 0,075 |
| Disteardimonium Hectorite | 0.6 | 0,53 | 0,3 | 0,53 |
| Butane + Isobutane + Propane | 85 | 85 | 85 | 85 |
| | | | | |
| Staining (Db*) | 6,1 | 4,9 | 3,9 | 4,9 |

Die Ergebnisse zeigen eine eindeutige Fleckenverminderung durch den Einsatz von Silikon-Gums.

Der Anteil an Aktivgehalt Silikon-Gum wird daher erfindungsgemäß im Bereich von 0,02 - 0,5 Gew.%, bezogen auf die Gesamtmasse der Zubereitung, besonders bevorzugt im Bereich von 0,035 % bis 0,2 Gew.%, insbesondere im Bereich von 0,04 bis 0,1 Gew.%, bezogen auf die Gesamtmasse der Zubereitung incl. Treibmittel, gewählt.

Als bevorzugtes Silikon-Gum wurde DC 1413-Fluid eingesetzt. DC 1413 umfasst 14% erfindungsgemäßes Silikon-Gum (Dimethicone; M>100.000 g/mol). Diese Mischung DC 1413 kann vorteilhaft zu 5 Gew.% eingesetzt werden, so dass sich der Aktivgehalt an Silikon-Gum zu 0,7 Gew.% in der Wirkstofflösung ohne Treibgas ergibt. Bei einem Treibgasanteil von ca. 85% ergibt sich dann ein vorteilhafter Anteilsbereich von ca. 0,105 Gew.% Silikon-Gum , bezogen auf die Gesamtmasse der Zubereitung inkl. Treibgas. In den Beispielzubereitungen sind 0,5 Gew.% (bzw. 0,8 Gew.%) DC 1413 eingesetzt worden, jeweils bereits bezogen auf die Gesamtmasse der Zubereitung incl. Treibmittel.

Damit liegt der Anteil an Si-Gum in der Gesamtzubereitung (Aktivgehalt) bei 0,07 Gew.% (bzw. 0,112 Gew.%).

Neben den dargestellten Untersuchungen zur Ermittlung des Gelbwertes b* wurde bei den verschiedenen Waschtests durch in Augenscheinnahme festgestellt, dass bei den erfindungsgemäßen Zubereitungen die gelben Flecken verschwunden bzw. vermindert waren.

In den durchgeführten und auch weiteren Untersuchungen wurde weiter festgestellt, dass auch eine spezielle Lipidkombination einen überraschenden Einfluss auf die Verminderung bzw. Vermeidung durch Antitranspirantwirkstoffe mitverursachten gelblicher textiler Flecken ausübt.

Beispiel B und C umfassen neben dem erfindungsgemäßen Silikon-Gum auch Isopropyl Palmitat bzw.C12-15 Alkyl Benzoat. Beide Lipide führen zu einer weiteren Gelbfleckenverminderung. Diese unterstützende Wirkung wurde bei weiteren bestimmten Lipiden festgestellt.

Erfindungsgemäß umfassen die Aerosolzubereitungen daher vorteilhaft ein oder mehrere Silikon-Gums und ein oder mehrere Lipide, gewählt aus der Gruppe Isopropylester, vorzugsweise Isopropylpalmitat, Alkylbenzoat, vorzugsweise C12-15 Alkylbenzoat, Triglyceride, vorzugsweise Caprylic/Capric Triglycerid, Dimethicone und/oder Kohlenwasserstoffgemische mit einer überwiegenden Kettenlänge ≥ C16.

Überwiegend bedeutet erfindungsgemäß, dass der Anteil an Bestandteilen im Kohlenwasserstoffgemisch mit Kettenlängen größer/gleich C16 mindestens 50%, bevorzugt mindestens 60%, beträgt. Kohlenwasserstoffgemische, die ausschließlich aus Bestandteilen mit C16 als Kettenlängen bestehen, sind demnach nicht erfindungsgemäß. Hierunter sind auch C14-22 Alkane möglich.

Ebenso bevorzugt ist ein Kohlenwasserstoff-Gemisch mit einer Kohlenstoffanzahl Cx mit x =16 + 4*a, wobei a aus der Menge der natürlichen Zahlen N = {0, 1, 2, 3,....} gewählt wird. Der Anteil an Kohlenwasserstoffen mit a = 0 beträgt mindestens 50 Gew.%, bevorzugt mindestens 55 Gew.%, insbesondere mindestens 60 Gew.%, bezogen auf die Gesamtmasse des Kohlenwasserstoffgemisches.

Das Kohlenwasserstoffgemisch Cx ist aus Oligomeren des Buten bzw. Butadien gebildet.

Erfindungsgemäß bevorzugt beträgt der Anteil an Kohlenwasserstoffen Cx mit a ≥ 1 mindestens 0,5 Gew.% und mit a > 4 (d.h. KW >C28) weniger als 0,5 Gew.%, jeweils bezogen auf die Gesamtmasse des Kohlenwasserstoffgemisches.

D.h. es sind mindestens 0,5 Gew.% an C20, C24, C28 oder C30 Kohlenwasserstoffen enthalten. Der Anteil an C32, C36 etc. beträgt weniger als 0,5 Gew.%.

Der Anteil an Bestandteilen im Kohlenwasserstoffgemisch Cx mit Kettenlängen gleich C16 beträgt mindestens 50%, bevorzugt mindestens 55%. Kohlenwasserstoffgemische, die ausschließlich aus Bestandteilen mit C16 als Kettenlängen bestehen, sind dabei nicht erfindungsgemäß bevorzugt.

Diese Lipide eignen sich bei der kombinierten Verwendung mit Silikon-Gums in ATzubereitungen zur Verminderung bzw. Vermeidung der durch die Zubereitung mit verursachten gelben Flecken auf oder in der Kleidung, insbesondere baumwollhaltige Kleidung.

Der Fachmann erwartete allerdings eine begrenzte Löslichkeit von Silikon-Gums in den bevorzugten nichtflüchtigen Ölen Isopropylpalmitat, Alkylbenzoat, Caprylic/Capric Triglycerid und/oder lineare/verzweigten Kohlenwasserstoffölen mit einer überwiegenden Kettenlänge ≥ C16. Jedoch ist es überraschender Weise gelungen, die Silikon-Gums stabil und wirksam einzuarbeiten. Erfindungsgemäß zeigen sich Kohlenwasserstoffe z. B. C14-22 Alkane mit überwiegender Kettenlänge ≥ C16 oder die bevorzugten KW-Gemische Cx darüber hinaus als kostengünstige Alternative.

Lipide bezeichnen Fette, fettähnliche Stoffe und Öle. Für die Kosmetik sind sie vor allem als weichmachende ("emollient") Inhaltsstoffe und als hauteigene Lipide der Hornschicht, die zwischen den Hornzellen lagern, von Bedeutung. Sie befähigen die Haut zur Speicherung von Feuchtigkeit. Neben dem pflegenden Aspekt werden Lipide den kosmetischen Zubereitungen zugesetzt, um eine bessere Verteilbarkeit auf der Haut zu gewährleisten und um die sensorischen Eigenschaften der Zubereitungen zu verbessern.

Durch den Einsatz dieser Lipide wird zum einen ein zusätzlich pflegendes Hautgefühl vermittelt, die gelben Flecken in oder auf der Kleidung vermindert und zudem die weißen Rückstände des Aluminiumchlorohydrates maskiert. Das bedeutet, dass sowohl weniger gelbe Flecken als auch weniger weiße Rückstände auf der Haut und schwarzer Kleidung sichtbar sind.

Die Zubereitungen umfassen mindestens ein antitranspirantwirksamen Stoff. Neben den eigentlichen schweißhemmenden Wirkstoffen (AT-Wirker) können zusätzlich auch Stoffe enthalten sein, die den mikrobiellen Abbau des Schweißes hemmen, wie z. B. Butyloctanoic Acid. Butyloctanoic Acid zeigt jedoch nur eine desodorierende Wirkung und keine antitranspirante Wirkung, da aus der Beeinflussung der bakteriellen Hautflora keine Beeinflussung der Schweißsekretion abzuleiten ist.

Insbesondere bei Antitranspirantien auf Basis von Aluminiumsalzen kann ggf. auf den weiteren Zusatz von rein desodorierend wirkenden Substanzen verzichtet werden, da diese per se ein antimikrobielles Potential aufweisen. Des Weiteren wird durch das reduzierte Wasserangebot durch die verminderte Schweißsekretion auch das bakterielle Wachstum gehemmt.

Antitranspirantwirkstoffe im Sinne der vorliegenden Anmeldung sind insbesondere aus den folgenden Gruppen zu wählen.

Als Antitranspirantwirkstoffe finden insbesondere Adstringenzien Verwendung, vornehmlich Aluminiumverbindungen. Die früher eingesetzten, stark sauer wirkenden Salze Aluminiumsulfat oder -chlorid und die Agaricinsäure sind weitgehend durch Aluminiumhydroxychlorid und - alkoholate ersetzt worden. Die nachfolgende Auflistung vorteilhaft einzusetzender Antitranspirant-Wirker soll in keinster Weise einschränkend sein:

### Aluminiumsalze:

- Aluminiumsalze wie Aluminiumchlorid AICI3, Aluminiumsulfat Al2(SO4)3
- Aluminiumchloride der empirischen Summenformel [Al2(OH)mCln], wobei m+n=6
- Aluminiumchlorhydrat [Al2(OH)5Cl] x H2O
   ▪ Standard Al-Komplexe: Locron P (Clariant), Micro-Dry 323 (Summit Reheis), Aloxicoll PF 40 (BK Giulini).
   ∘ Aktivierte Al-Komplexe: Reach 103 (Summit Reheis), AACH-7171/AACH-7172 (Summit Reheis), Aloxicoll P (BK Giulini), Aloxicoll SD100 (BK Giulini)
- Aluminiumsesquichlorhydrat [Al2(OH)4,5Cl1,5] x H2O Standard Al-Komplexe: Aloxicoll 31P (BK Giulini)
- Aluminiumdichlorhydrat [Al2(OH)4Cl2] x H2O

Die Aluminiumsalze können in gemahlener Form oder auch als Hohlkugeln oder als Mischung dieser vorliegen. Die Dichte dieser Partikel liegt vorteilhaft im Bereich von 0,7 bis 2,0 g/cm³.

Die Antitranspirantwirkstoffe aus den zuvor geschilderten Gruppen werden in den Formulierungen bevorzugt in einer Menge von 0,5 bis 10 Gew.%, bevorzugt 2 bis 6 Gew.%, bezogen auf die Gesamtmasse der Zubereitung, d.h. inklusive der vorhandenen Treibgase, eingesetzt.

Bei Einsatz von ca. 35 Gew.% AACH (aktiviertes Aluminiumchlorohydrat) in der Wirkstofflösung für ein Aerosolspray (ohne Treibgas) und einem Abfüllverhältnis von etwa 15 : 85 (Wirkstofflösung zu Treibgas) wird ein Anteil von etwa 5,25 Gew. % AACH im Endprodukt vorhanden sein.

Der Anteil an Aluminiumsalzen wird vorteilhaft im Bereich von 1,0 bis 6 Gew.%, bevorzugt 1,0 bis 3,0 Gew.%, bezogen auf die Gesamtmasse der Zubereitung, gewählt.

Zur Reduzierung der AT-Mittel induzierten Textilverfleckung kann es erfindungsgemäß vorteilhaft sein, die Einsatzkonzentration des AT-Wirkstoffes im Vergleich zu einem Standard-Produkt zu reduzieren. Die untere Grenze der erfindungsgemäß vorteilhaften Einsatzkonzentration wird durch die Maßgabe bestimmt, dass eine antitranspirante Wirkung mit dem Produkt realisiert sein muss.

Der erfindungsgemäße Zusatz an Silikon-Gums ermöglicht erstaunlicherweise die Reduzierung an Aluminiumsalzen. Durch die Reduzierung des AT-Mittels wird das Verhältnis zwischen Si-Gum und AT-Mittel erfindungsgemäß vorteilhaft beeinflusst. Eine Reduzierung der Gelbverfleckung konnte bereits bei einem Verhältnis Si-Gum (Aktivgehalt) zu AT-Mittel von weniger als 1:100 nachgewiesen werden. Ein vorteilhaftes Verhältnis zwischen Si-Gum und AT-mittel liegt bei etwa 1:75, überaus deutliche Effekte wurden bei einem Verhältnis von ca. 1:20 gefunden.

Damit ist es erfindungsgemäß vorteilhaft das Gewichtsverhältnis von Silikon-Gums zu Antitranspirantmitteln, insbesondere Aluminiumsalzen, insbesondere Aluminiumchlorohydrate, im Bereich von 1 zu 100 bis 1:10, insbesondere 1 zu 75 bis 1 zu 15, insbesondere 1 zu 20, zu wählen.

Bevorzugt werden in Antitranspirant-Aerosolzubereitungen Aluminiumchlorohydrate, insbesondere aktivierte Aluminiumchlorohydrate (AACH), als AT-Wirker eingesetzt.
Im erfindungsgemäßen Aerosol wird als Treibgas bevorzugt Propan, Propen, n-Butan, iso-Butan, iso-Buten, n-Pentan, n-Penten, iso-Pentan, iso-Penten, Methan und Ethan einzeln oder in Kombination eingesetzt. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt.

Besonders bevorzugt sind Propan, Butan, iso-Butan bzw. Mischungen dieser Treibgase. Die genannten Gase können im Sinne der vorliegenden Erfindung jeweils einzeln oder in beliebigen Mischungen zueinander verwendet werden. Bevorzugt werden Mischungen mit Druckstufen von 1,0 bis 3,5 bar eingesetzt. Bevorzugt 2,5 bis 3,0, ganz besonders bevorzugt ist eine Druckstufe von 2,7 bar.

Bevorzugte Abfüllverhältnisse von Wirkstofflösung zu Treibgas betragen 40:60 bis 5 :95, bevorzugt 25:75 bis 10:90, ganz besonders bevorzugt 15:85.

Die Zubereitungen sind vorteilhaft frei von Tensiden, Emulgatoren, Antioxidantien und/oder Komplexbildnern. Vorteilhaft sind die Zubereitungen sowohl frei von Tensiden, frei von Emulgatoren, frei von Antioxidantien und frei von Komplexbildnern.

Insbesondere sind die Zubereitungen frei von Butylhydroxytoluol (BHT), Pentaerythrityl tetra-di-t-butyl hydroxyhydrocinnamat (TTHP, BHPM), Coffein, Abies Picea Extract und Zitronensäure.

"Frei von" bedeutet dabei, dass der Anteil dieser Stoffe in der Zubereitung 0 Gew.%, bezogen auf die Gesamtmasse der Zubereitung beträgt, abgesehen von Verunreinigungen oder anderweitigen unbeabsichtigten Einschleppungen, beispielsweise durch verunreinigte Rohstoffe oder bei der Herstellung.

Ebenso ist es von Vorteil, wenn die Zubereitungen frei von bestimmten Maskierungsmitteln, wie insbesondere Phenyl Trimethicone und/oder Triethylcitrate, sind.

Phenyl Trimethicone sind u.a. zur Maskierung weißer Rückstände bekannt. Weiterhin soll Phenyl Trimethicon zwar die Fähigkeit besitzen, die Bildung gelber Flecken durch AT-Mittel zu verringern. Diese Eigenschaften sind im Stand der Technik beschrieben und werden für Nicht-Aerosol-Anwendungen offenbart. Die Anwendung von Phenyl Trimethicone für Aerosolprodukte ist jedoch unter Berücksichtigung toxikologischer Aspekte nicht uneingeschränkt zu empfehlen.

Die vorliegende Erfindung der Verwendung von Silikon-Gums geht daher einen anderen Weg unerwünschte gelbe Flecken zu vermindern bzw. zu vermeiden.

Triethylcitrat ist als Antioxidanz, Deo-Wirker, Maskierungsöl und Lösungsmittel beschrieben. Aufgrund seiner Polarität kommt es insbesondere in Parfümölen als Lösungsmittel zum Einsatz. Die Formulierung größerer Mengen an Triethylcitrat kann aber bei Trägersystemen mit vorwiegend unpolaren Trägerölen galenische Schwierigkeiten bereiten.

Aufgrund dessen ist der Zusatz an Triethylcitrat erfindungsgemäß vorteilhaft zu vermeiden.

Die Aerosolzubereitungen können kosmetische Hilfsstoffe und weitere Wirkstoffe enthalten, wie sie üblicherweise in Antitranspirantzubereitungen verwendet werden, wie z. B. Konservierungsmittel, Konservierungshelfer, Bakterizide, Parfüme, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften nicht beeinträchtigt oder erfindungsgemäß ausgeschlossen ist.

Bevorzugte Applikationsform für die Formulierungen sind wasserfreie Aerosole, die in Aluminiumdosen auf den Markt kommen. Bevorzugt ist diese Applikationsform, da sie weltweit die häufigste Form im Bereich Deo/AT darstellt. Auch aufgrund der sehr guten Packmittelkompatibilität ist ein Einsatz in wasserfreien Formulierungen zu empfehlen, da es auch nach Lagerung bei +40°C über einen Zeitraum von 6 Monaten kaum zu Packmittelveränderungen kommt.

Die nachfolgenden Beispiele illustrieren Zubereitungen. Die Angaben sind Gewichtsanteile, bezogen auf die Gesamtmasse der jeweiligen Zubereitungen inkl. Treibgas.

### Beispiele

| INCI | 1 | 2 |
|---|---|---|
| Aluminum Chlorohydrate (Activated) | 5.25 | 2,5 |
| Cyclomethicone | ad 100 | ad 100 |
| Dimethicone (86% M<100,000) + Dimethicone (14%) | 0.5 (0.07 % Aktivgehalt Silikon-Gum) | 0,8 (0,112) |
| Octyldodecanol | 0,1 | 0,1 |
| Dimethicone | 0.45 | --- |
| Isopropyl Palmitate | 1,5 | 1,5 |
| C12-15 Alkyl Benzoate | --- | 1,5 |
| Parfum | 1,0 | 1,0 |
| Propylene Carbonate | 0,075 | 0,1 |
| Disteardimonium Hectorite | 0,53 | 0,3 |
| Butane + Isobutane + Propane | 85 | 85 |
| | | |
| Staining (Db*) | 4,9 | 3,9 |

In der nachfolgenden Liste sind die verwendeten Rohstoffe und deren Handelsbezeichnungen benannt:

| INCI | Handelsname | Hersteller/Lieferant |
|---|---|---|
| Aluminum Chlorohydrate | Aloxicoll PF 40 | BK Giulini Chemie |
| Butyloctanoic Acid | Isocarb 12 | Sasol |
| Aluminum Chlorohydrate (Activated) | Activated Aloxicoll P | BK Giulini Chemie |
| Cyclomethicone | Baysilone SF 1202 | Momentive Performance Materials Inc, |
| Persea Gratissima Oil | Avocado Oil, raff,DAC | Henry Lamotte |

| Isopropyl Palmitate | Isopropylpalmitate | Cognis |
|---|---|---|
| Cyclomethicone + Dimethiconol | DC 1501 Fluid | Dow Corning |
| Cyclomethicone + Dimethicone | DC 1411 Fluid | Dow Corning |
| Dimethicone + Dimethiconol | DC 1503 Fluid | Dow Corning |
| C13-16 Isoparaffin + Dimethicone + C10-13 Isoparaffin | DC BY 25-320 | Dow Corning |
| Dimethicone + Dimethicone | DC 1413 Fluid | Dow Corning |
| C12-15 Alkyl Benzoate | Tegosoft TN 2 | Evonik Goldschmidt |
| Octyldodecanol | Isofol 20 | Sasol |
| Dimethicone (M <100,000 g/mol) | BRB Silikone Oil DM 100 | BRB International |
| Propylene Carbonate | 807051 Propylencarbonat zur Synthese | Merck |
| Disteardimonium Hectorite | Bentone 38V CG | Elementis |
| Butane + Isobutane + Propane | Drivosol 27 D60 | Evonik Oxeno |

Die Beispiele zeigen, dass die Zubereitungen nur die erfindungsgemäßen Silikon-Gums, antitranspirantwirksame Stoffe, insbesondere Aluminiumsalzen, die vorteilhaft genannten Lipiden, Treibmitteln und flüchtigen Silikonölen und lediglich noch Schichtsilikate, Propylencarbonat, sowie ggf. Hautpflegemittel (Emollients) und/oder desodorierende Stoffe (Deodorantien), wie beispielsweise Parfüm, umfassen.
Die kosmetischen Aerosolzubereitungen bestehen daher vorteilhaft lediglich aus ein oder mehreren Silikon-Gums, ein oder mehreren antitranspirant wirksamen Stoffen, insbesondere Aluminiumsalzen, ein oder mehrere Lipide, gewählt aus der Gruppe Isopropylester, vorzugsweise Isopropylpalmitat, Alkylbenzoat, vorzugsweise C12-15 Alkylbenzoat, Triglyceride, vorzugsweise Caprylic/Capric Triglycerid, Dimethicone und/oder Kohlenwasserstoffgemische mit einer überwiegenden Kettenlänge ≥ C16, ein oder mehrere Treibmittel, ein oder mehrere bei Raumtemperatur flüchtige Silikonöle, insbesondere Cyclomethiconen, ein oder mehrere Schichtsilikate, insbesondere Disteardimonium Hectorit, Propylencarbonat, ggf. Hautpflegemittel und Parfum.

Diese Zubereitungen weisen ein hervorragende AT-wirksamkeit auf, sind hautverträglich und vor allem werden gelbe Flecken in oder auf der Kleidung vermieden bzw. vermindert.

## Patentansprüche

1. Verwendung von ein oder mehreren Silikon-Gums in kosmetischen oder dermatologischen Zubereitungen umfassend ein oder mehrere antitranspirantwirksame Stoffe zur Verminderung oder Vermeidung der Fleckenbildung in oder auf der Kleidung **dadurch gekennzeichnet, dass** als Silikon-Gums Polydiorganosiloxane, Dimethicone und Dimethiconol mit einer Molmasse im Bereich von 100.000 bis 2.000.000 g/mol gelten.

2. Verwendung von ein oder mehreren Silikon-Gums in kosmetischen oder dermatologischen Zubereitungen umfassend ein oder mehrere antitranspirantwirksame Stoffe zur Verbesserung der Auswaschbarkeit von Flecken, die durch die Zubereitung mit verursacht sind, in oder auf der Kleidung **dadurch gekennzeichnet, dass** als Silikon-Gums Polydiorganosiloxane, Dimethicone und Dimethiconol mit einer Molmasse im Bereich von 100.000 bis 2.000.000 g/mol gelten.

3. Verwendung nach Anspruch 1 oder 2 **dadurch gekennzeichnet, dass** der Anteil an Silikon-Gum im Bereich von 0,02 bis 0,5 Gew.%, bezogen auf die Gesamtmasse der Zubereitung, gewählt wird.

4. Verwendung nach Anspruch 3 **dadurch gekennzeichnet, dass** der Anteil an Silikon-Gum im Bereich von 0,035 % bis 0,2 Gew.%, bezogen auf die Gesamtmasse der Zubereitung, gewählt wird.

5. Verwendung nach einem der vorstehenden Ansprüche **dadurch gekennzeichnet, dass** die Zubereitungen wasserfrei sind.

6. Verwendung nach einem der vorstehenden Ansprüche **dadurch gekennzeichnet, dass** die Zubereitung ein oder mehrere Lipide, gewählt aus der Gruppe Isopropylester, vorzugsweise Isopropylpalmitat, Alkylbenzoat, vorzugsweise C12-15 Alkylbenzoat, Triglyceride, vorzugsweise Caprylic/Capric Triglycerid, Dimethicone und/oder Kohlenwasserstoffgemische mit einer überwiegenden Kettenlänge ≥C16 umfasst.

7. Verwendung nach einem der vorstehenden Ansprüche **dadurch gekennzeichnet, dass** die Zubereitung ein oder mehrere Lipide, gewählt aus der Gruppe Isopropylpalmitat, C12-15 Alkylbenzoat, Caprylic/Capric Triglycerid, Dimethicon und/oder Kohlenwasserstoffgemische mit einer überwiegenden Kettenlänge ≥C16 umfasst.

8. Verwendung nach einem der vorstehenden Ansprüche **dadurch gekennzeichnet, dass** als antitranspirantwirksame Stoffe ein oder mehrere Aluminiumsalze, insbesondere Aluminiumchlorohydrate, gewählt werden.

9. Verwendung nach Anspruch 8 **dadurch gekennzeichnet, dass** der Anteil an Aluminiumsalzen im Bereich von 1,0 bis 6 Gew.%, bevorzugt 1,0 bis 3,0 Gew.%, bezogen auf die Gesamtmasse der Zubereitung gewählt werden.

10. Verwendung nach einem der vorstehenden Ansprüche **dadurch gekennzeichnet, dass** die Zubereitungen frei von Tensiden, Emulgatoren, Antioxidantien und/oder Komplexbildnern sind.

11. Verwendung nach einem der vorstehenden Ansprüche **dadurch gekennzeichnet, dass** die Zubereitungen frei von Butylhydroxytoluol (BHT), Pentaerythrityl tetra-di-t-butyl hydroxyhydrocinnamat (TTHP, BHPM), Coffein, Abies Picea Extract, Zitronensäure, Phenyl Trimethicon und/oder Triethylcitrat sind.

12. Verwendung nach einem der vorstehenden Ansprüche **dadurch gekennzeichnet, dass** das Gewichtsverhältnis von Silikon-Gums zu antitranspirantwirksamen Stoffen im Bereich von 1 zu 100 bis 1 zu 10, insbesondere 1 zu 75 bis 1 zu 15 gewählt wird.

## Claims

1. Use of one or more silicone gums in cosmetic or dermatological preparations comprising one or more antiperspirant active ingredients for reducing or preventing staining in or on clothing, **characterized in that** polydiorganosiloxanes, dimethicone and dimethiconol having a molar mass in the range of 100 000 to 2 000 000 g/mol are effective as silicone gums.

2. Use of one or more silicone gums in cosmetic or dermatological preparations comprising one or more antiperspirant active ingredients for improving the washability of stains, which are aggravated by the preparation, in or on clothing, **characterized in that** polydiorganosiloxanes, dimethicone and dimethiconol having a molar mass in the range of 100 000 to 2 000 000 g/mol are effective as silicone gums.

3. Use according to Claim 1 or 2, **characterized in that** the proportion of silicone gum is selected in the range of 0.02 to 0.5% by weight, based on the total mass of the preparation.

4. Use according to Claim 3, **characterized in that** the proportion of silicone gum is selected in the range of 0.035% to 0.2% by weight, based on the total mass of the preparation.

5. Use according to any of the preceding claims, **characterized in that** the preparations are free of water.

6. Use according to any of the preceding claims, **characterized in that** the preparation comprises one or more lipids selected from the group comprising isopropyl esters, preferably isopropyl palmitate, alkyl benzoate, preferably C12-15 alkyl benzoate, triglycerides, preferably caprylic/capric triglyceride, dimethicone and/or hydrocarbon mixtures having a predominant chain length ≥C16.

7. Use according to any of the preceding claims, **characterized in that** the preparation comprises one or more lipids selected from the group comprising isopropyl palmitate, C12-15 alkyl benzoate, caprylic/capric triglyceride, dimethicone and/or hydrocarbon mixtures having a predominant chain length ≥ C16.

8. Use according to any of the preceding claims, **characterized in that** one or more aluminium salts, especially aluminium chlorohydrate, are selected as antiperspirant active ingredients.

9. Use according to Claim 8, **characterized in that** the proportion of aluminium salts are selected in the range of 1.0 to 6% by weight, preferably 1.0 to 3.0% by weight, based on the total mass of the preparation.

10. Use according to any of the preceding claims, **characterized in that** the preparations are free of surfactants, emulsifiers, antioxidants and/or chelating agents.

11. Use according to any of the preceding claims, **characterized in that** the preparations are free of butylhydroxytoluene (BHT), pentaerythrityl tetra-di-t-butyl hydroxyhydrocinnamate (TTHP, BHPM), caffeine, Picea abies extract, citric acid, phenyl trimethicone and/or triethyl citrate.

12. Use according to any of the preceding claims, **characterized in that** the weight ratio of silicone gums to antiperspirant active ingredients is selected in the range from 1:100 to 1:10, especially 1:75 to 1:15.

## Revendications

1. Utilisation d'une ou de plusieurs gommes de silicone dans des préparations cosmétiques ou dermatologiques comprenant une ou plusieurs substances à effet anti-transpirant pour réduire ou éviter la formation de taches dans ou sur les vêtements, **caractérisée en ce que** des polydiorganosiloxanes, des diméthicones et du diméthiconol ayant une masse molaire dans la plage allant de 100 000 à 2 000 000 g/mol sont utilisés en tant que gommes de silicone.

2. Utilisation d'une ou de plusieurs gommes de silicone dans des préparations cosmétiques ou dermatologiques comprenant une ou plusieurs substances à effet anti-transpirant pour améliorer l'aptitude au lavage de taches causées par la préparation dans ou sur les vêtements, **caractérisée en ce que** des polydiorganosiloxanes, des diméthicones et du diméthiconol ayant une masse molaire dans la plage allant de 100 000 à 2 000 000 g/mol sont utilisés en tant que gommes de silicone.

3. Utilisation selon la revendication 1 ou 2, **caractérisée en ce que** la proportion de gomme de silicone est choisie dans la plage allant de 0,02 à 0,5 % en poids, par rapport à la masse totale de la préparation.

4. Utilisation selon la revendication 3, **caractérisée en ce que** la proportion de gomme de silicone est choisie dans la plage allant de 0,035 à 0,2 % en poids, par rapport à la masse totale de la préparation.

5. Utilisation selon l'une quelconque des revendications précédentes, **caractérisée en ce que** les préparations sont anhydres.

6. Utilisation selon l'une quelconque des revendications précédentes, **caractérisée en ce que** la préparation comprend un ou plusieurs lipides, choisis dans le groupe constitué par les esters isopropyliques, de préférence le palmitate d'isopropyle, le benzoate d'alkyle, de préférence le benzoate d'alkyle en C12-15, les triglycérides, de préférence le triglycéride caprylique/caprique, les diméthicones et/ou les mélanges d'hydrocarbures ayant une longueur de chaîne principale ≥ C16.

7. Utilisation selon l'une quelconque des revendications précédentes, **caractérisée en ce que** la préparation comprend un ou plusieurs lipides, choisis dans le groupe constitué par le palmitate d'isopropyle, le benzoate d'alkyle en C12-15, le triglycéride caprylique/caprique, la diméthicone et/ou les mélanges d'hydrocarbures ayant une longueur de chaîne principale ≥ C16.

8. Utilisation selon l'une quelconque des revendications précédentes, **caractérisée en ce qu'**un ou plusieurs sels d'aluminium, notamment des chlorohydrates d'aluminium, sont choisis en tant que substances à effet anti-transpirant.

9. Utilisation selon la revendication 8, **caractérisée en ce que** la proportion de sels d'aluminium est choisie dans la plage allant de 1,0 à 6 % en poids, de préférence de 1,0 à 3,0 % en poids, par rapport à la masse totale de la préparation.

10. Utilisation selon l'une quelconque des revendications précédentes, **caractérisée en ce que** les préparations sont exemptes de tensioactifs, d'émulsifiants, d'antioxydants et/ou de complexants.

11. Utilisation selon l'une quelconque des revendications précédentes, **caractérisée en ce que** les préparations sont exemptes de butylhydroxytoluène (BHT), d'hydroxyhydrocinnamate de pentaérythrityl-tétra-di-t-butyle (TTHP, BHPM), de caféine, d'extrait d'Abies Picea, d'acide citrique, de phényltriméthicone et/ou de citrate de triéthyle.

12. Utilisation selon l'une quelconque des revendications précédentes, **caractérisée en ce que** le rapport en poids entre les gommes de silicone et les substances à effet anti-transpirant est choisi dans la plage allant de 1 sur 100 à 1 sur 10, notamment de 1 sur 75 à 1 sur 15.
